# EUROPEAN PATENT APPLICATION

(11) **EP 4 361 130 A1**
(43) Date of publication of application: **01.05.2024**
(21) Application number: 22383049.8
(22) Date of filing: 31.10.2022
(51) Int. Cl.: C07D 209/20, C07C 319/28, C12P 13/12, C12P 13/22

(54) **METHOD OF TREATING A CHEMICAL PRODUCT**

(71) Applicant: Illinois Tool Works Inc., Glenview, IL 60025 (US)
(72) Inventor: ONDONO MOLINA, Raul, Glenview, 60025 (US); TORRALVO MARTIN, Hector, Glenview, 60025 (US); FERNANDEZ FREIXES, Guillem, Glenview, 60025 (US); MARTIN SANCHEZ, Laura, Glenview, 60025 (US)
(74) Representative: HGF

(57) **Abstract**

Provided is method of treating a product of a chemical process, such as an amino acid or a nucleoside, the method comprising adding the product to a solvent such as propanol in a vessel to obtain a mixture, stirring the mixture, filtering the mixture so as to separate the product from the solvent and drying the filtered product.

## Description

### Field of the invention

The present invention relates to a method of treating a product of a chemical process, in particular to a method of treating a product with a solvent, such as propanol.

### Background to the invention

Amino acids are used as raw materials in a wide variety of industries, including industrial processes, food products for animal and human consumption, pharmaceuticals and cosmetics.

Raw materials such as amino acids are typically available in different qualities or 'grades', including feed grade, food grade and pharmaceutical grade. Feed grade refers to products that are suitable for animal (e.g. pets, livestock and zoo animals) consumption. Food grade refers to products that meet the standards for safe human consumption. Generally, food grade products will have fewer impurities and microorganisms than feed grade. Pharmaceutical grade products are highly pure and meet the pharmaceutical standards for manufacturing. In Europe, the quality standard for medicines and the materials used to make them is described by the European Pharmacopoeia.

Due to their higher quality, pharmaceutical grade raw materials tend to be costly compared to food grade or feed grade products. Furthermore, for some raw materials, only a few manufacturers may supply the material in pharmaceutical grade, resulting in a weak supply chain. It order to reduce reliance on expensive products which are only available from a limited number of suppliers, it is often desirable for manufacturers to be able to purchase lower grade products and then purify them to produce a higher grade product. It is also desirable for manufacturers to be able to purify raw materials, such as amino acids, to ensure that they meet the relevant standards, such as those determined by the European Pharmacopoeia.

The present invention has been devised with these issues in mind.

### Summary of the invention

According to a first aspect of the invention there is provided a method of treating a product of a chemical process, the method comprising:
- adding the product to a solvent in a vessel to obtain a mixture;
- stirring the mixture;
- filtering so as to separate the product from the solvent;
- drying the filtered product.

According to a second aspect of the invention there is provided a method of preparing a product (e.g. a chemical product), the method comprising:
- synthesising the product (e.g. via chemical synthesis), purifying the product, modifying the product, and/or forming a salt of the product; and
- treating the product according to the method of the first aspect of the invention.

According to a third aspect of the invention there is provided the use of propanol (e.g. 2-propanol), subsequent to a chemical process for synthesising, purifying and/or modifying a product and/or forming a salt of the product, for:
- decolouring the product;
- reducing the bioburden of the product and/or inhibiting the growth of microorganisms in the product; and/or
- improving the crystallinity of the product.

According to a fourth aspect of the invention, there is provided a method for preparing an amino acid, a nucleoside, or a salt thereof, comprising the method of the first aspect of the invention.

### Detailed description

Embodiments of the invention will now be described by way of example with reference to the accompanying drawings in which:
Figure 1 shows a process for treating L-cystine dihydrochloride with 2-propanol;
Figure 2 shows a process for treating L-tryptophan with 2-propanol; and
Figure 3 shows a process for treating *N*-acetyl tryptophan with 2-propanol.

The present invention provides a method of treating a product of a chemical process, the method comprising:
- adding the product to a solvent in a vessel to obtain a mixture;
- stirring the mixture;
- filtering so as to separate the product from the solvent; and
- drying the filtered product.

In some embodiments, the solvent is an organic polar solvent. The solvent may be selected from an alcohol (e.g. methanol, ethanol, or propanol), acetonitrile or acetone, or a mixture thereof. In some embodiments, the solvent is an alcohol. The alcohol may be methanol, ethanol, or propanol (e.g. 1-propanol or 2-propanol). In some embodiments, the solvent is 2-propanol (also known as isopropanol).

Advantageously, it has been found that treatment of a product, such as an amino acid or a nucleoside, with propanol improves the quality of the product in terms of both its colour and crystallinity. Propanol treatment has been found to result in a whiter solid having a greater level of crystallinity, and a clearer solution of the product, versus products which have not been subjected to the propanol treatment.

The chemical process may comprise or consist of a process for synthesising the product, a process for purifying the product, a process for modifying the product (e.g. chemical modification or racemization), and/or a process for forming a salt of the product.

The product may be selected from the group consisting of an amino acid, a nucleoside or a salt and/or dimer thereof.

The amino acid may be a proteinogenic, standard, non-standard, natural or non-natural amino acid. In some embodiments, the amino acid is selected from: arginine, histidine, lysine, aspartic acid, glutamic acid, serine, threonine, asparagine, glutamine, glycine, proline, cysteine, alanine, valine, isoleucine, leucine, methionine, phenylalanine, tyrosine, tryptophan, selenocysteine, pyrrolysine, ornithine, norleucine, t-leucine, norvaline, γ-aminobutyric acid (GABA), δ-aminolevulinic acid, δ-aminobenzoic acid (PABA), β-alanine, aminoisobutyric acid, cystathionine, lanthionine, djenkolic acid, diaminopimelic acid, α-amino-n-butyric acid, alloisoleucine, α-amino-n-heptanoic acid, pipecolic acid, α,β-diaminopropionic acid, α,γ-diaminobutyric acid, allothreonine, homoalanine, homocysteine, homoserine, isoserine, β-amino-n-butyric acid, β-aminoisobutyric acid, γ-aminobutyric acid, α-aminoisobutyric acid, isovaline, and α-hydroxy-γ-aminobutyric acid.

In some embodiments the amino acid is a proteinogenic amino acid, or a modified proteinogenic amino acid (e.g. an acetylated or a phosphorylated proteinogenic amino acid). Thus, in some embodiments the amino acid is selected from arginine, histidine, lysine, aspartic acid, glutamic acid, serine, threonine, asparagine, glutamine, glycine, proline, cysteine, alanine, valine, isoleucine, leucine, methionine, phenylalanine, tyrosine, tryptophan, selenocysteine and pyrrolysine. The proteinogenic amino acid may be modified.

The amino acid may be an L-amino acid or a D-amino acid (i.e. wherein the stereogenic carbon alpha to the amino group has the L- or D-configuration, respectively). In some embodiments, the product is a mixture (e.g. a racemic mixture) of the L- and D-configuration.

In some embodiments, the amino acid is an L-amino acid (e.g. L-tryptophan) or a salt thereof.

In some embodiments the product is an amino acid dimer (i.e. a molecule formed from two amino acid residues) or a salt thereof. In some embodiments the product is cystine (a dimer of two cysteine residues) or a salt thereof. The product may be L-cystine, or a salt thereof e.g. L-cystine dihydrochloride.

As used herein, the term "amino acid" includes modified amino acids in which a naturally occurring amino acid has been chemically altered, for example to include a side chain or other functional group. Examples of modified amino acids include amino acids which have been acylated (e.g. acetylated), alkylated (e.g. methylated), phosphorylated or hydroxylated. In some embodiments, the amino acid is an acetylated amino acid, such as *N*-acetyl tryptophan (e.g. NADLT), *N*-oleoylphenylalanine, *N*-stearoyltyrosine, *N*-ethylglycine, *N*-propylglycine, *N-*isopropylglycine, *N*-methylalanine, *N*-ethylalanine, *N-*methyl-β-alanine, *N*-ethyl-β-alanine, *O*-phospho-L-tyrosine, or *O*-phospho-L-serine.

It will therefore appreciated that references herein to "modifying" a product (e.g. an amino acid) refers to chemically altering the structure of the product through, for example, acylation (e.g. acetylation), alkylation (e.g. methylation), phosphorylated or hydroxylation.

The salt may be any suitable salt, such as a hydrochloride salt (e.g. mono- or dihydrochloride), a sodium salt, or a potassium salt.

In some embodiments, the product is selected from tryptophan (e.g. L-tryptophan), cystine (e.g. L-cystine dihydrochloride) and N-acetyl tryptophan (e.g. NADLT).

The nucleoside may be adenosine, guanosine, 5-methyluridine, uridine or cytidine. In some embodiments the nucleoside is guanosine.

In some embodiments, the method comprises adding the product, when wet, to the solvent. For example, a product of a synthetic process which contains water or another solvent may be transferred directly to the vessel without first drying. Alternatively, the method may comprise adding dry product to the solvent.

The solvent (e.g. 2-propanol) may be added to the vessel in an amount which is sufficient to ensure adequate stirring, but which is minimised in order to avoid loss of the product due to dissolution. For example, the amount of solvent may be from 0.5 kg to 5 kg solvent per kg of product.

The solvent (e.g. 2-propanol) may be substantially pure. For example, the solvent (e.g. 2-propanol) may be at least 95%, at least 96%, at least 97%, at least 98%, at least 99%, at least 99.5%, at least 99.6%, at least 99.7%, at least 99.8%, or at least 99.9% pure.

Any suitable vessel may be used. The vessel may have a capacity of from 0.5 L to 6000 L, depending on the amount of product to be treated. The vessel may be jacketed for controlling the temperature of the mixture.

In some embodiments, stirring is carried out at a temperature of from 5 to 80 °C, from 10 to 70 °C, from 15 to 60 °C, from 20 to 50 °C or from 30 to 40 °C.

In some embodiments, stirring comprises a first stirring step which is carried out at a first stirring temperature, and a second stirring step which is carried out at a second stirring temperature, wherein the second stirring temperature is lower than the first stirring temperature.

The first stirring temperature may be from 40 to 80 °C, or from 50 to 70 °C, e.g. about 60 °C. The second stirring temperature may be from 5 to 50 °C, from 10 to 40 °C or from 15 to 30 °C.

Thus, the method may further comprise heating the mixture, before or during stirring.

The method may further comprise cooling the mixture, before, during or after stirring.

The first stirring step may be carried out for a period of time of from 30 minutes to 4 hours, or from 1 to 3 hours, e.g. 2 hours.

The second stirring step may be carried out for a period of time of from 30 minutes to 4 hours, or from 1 to 3 hours, e.g. 2 hours.

For example, the method may comprise, after mixing the product with the solvent:
- heating the mixture to a first stirring temperature (e.g. 50 to 70 °C) and holding the mixture at the first temperature for a first period of time (e.g. 1 to 3 hours) while stirring; and
- reducing the temperature of the mixture to a second stirring temperature (e.g. 10 to 30 °C) and holding the mixture at the second temperature for a second period of time (e.g. 1 to 3 hours) while stirring.

Filtering may be carried out via any suitable means, such as centrifugation or by using a Büchner funnel.

Drying may be carried by heating the product, e.g. in an oven. In some embodiments, the product is dried by heating at a temperature that is less than 100 °C, less than 95 °C, less than 90 °C or less than 85 °C. The product may be dried by heating at a temperature of from 40 to 100 °C, from 50 to 90 °C or from 60 to 85 °C or from 70 to 80 °C.

In some embodiments, drying comprises a first drying step which is carried out at a first drying temperature, and a second drying step which is carried out at a second drying temperature, wherein the second drying temperature is higher than the first drying temperature.

The first drying temperature may be from 30 to 70 °C, from 35 to 60 °C or from 40 to 50 °C.

The second drying temperature may be from 50 to 100 °C, from 60 to 90 °C or from 70 to 80 °C.

The first drying step may be carried out for a period of time of from 30 minutes to 24 hours, from 1 to 20 hours, from 2 to 18 hours, from 4 to 16 hours, from 6 to 12 hours or from 8 to 10 hours.

The second drying step may be carried out for a period of time of from 30 minutes to 8 hours, from 1 to 6 hours, or from 2 to 5 hours e.g. 3-4 hours.

In some embodiments, the second drying step is shorter in duration than the first drying step.

In some embodiments, the first drying step is carried out at a lower temperature, and for a longer period of time, than the second drying step.

Advantageously, it has been found that drying the product for longer at a longer temperature, followed by a shorter drying step at a higher temperature, helps to minimize discolouration of the product.

For example, after filtering the product, the method may comprise:
- drying the product (e.g. in an oven) at a first drying temperature (e.g. from 40 to 60 °C) for a first period of time (e.g. 4 to 16 hours); and
- further drying the product (e.g. in an oven) at a second drying temperature (e.g. from 70 to 100 °C) for a second period of time (e.g. 1 to 2 hours).

Alternatively, in some embodiments the method comprises a single drying step. In such embodiments, drying may be carried out for a period of time of from 1 to 24 hours, from 2 to 20 hours, or from 4 to 18 hours (e.g. about 12 to 16 hours).

The method may further comprise monitoring the moisture content of the product during the drying step. For example, the moisture content of the product may be determined at regular intervals (e.g. every 30 minutes). This helps to ensure that the product is not exposed to the high temperatures used for drying for any longer than necessary.

The method may further comprise one or more additional steps selected from: sieving, weighing, packaging, tempering, inspecting, and/or quality control.

In some embodiments the chemical process comprises synthesising the product. The product may be synthesised by reacting two or more reactants.

For example, in embodiments wherein the product is *N*-acetyl tryptophan (e.g. NADLT), the chemical process may comprise synthesising the *N*-acetyl tryptophan from L-tryptophan and acetic anhydride. A process for the preparation of NADLT is described in J. Biol. Chem 1932, 96:511-517.

In some embodiments the chemical process comprises purifying the product. For example, a low-grade product (e.g. feed grade) may be purified so as to obtain a higher-grade product (e.g. food grade or pharma grade). The purified product may then be treated according to the method of the invention.

In some embodiments, purifying the product comprises recrystallisation.

For example, in embodiments wherein the product is an amino acid (such as tryptophan, e.g. L-tryptophan), the amino acid may be purified by recrystallisation prior to treatment according to the first aspect of the present invention. Processes for recrystallisation will be known to those skilled in the art, such as the method described in US5057615.

In some embodiments the chemical process comprises forming a salt. For example, the chemical process may comprise forming a salt, e.g. a salt of an amino acid. The salt may be a hydrochloride salt, such as a hydrochloride salt of cystine (e.g. L-cystine). A process for the preparation of L-cystine dihydrochloride is described in J. Am. Chem. Soc. 1958, 80: 3835-3838.

Thus, the invention also provides a method of preparing a product (e.g. a chemical product), the method comprising:
- synthesising (e.g. via chemical synthesis) and/or purifying the product (e.g. via recrystallisation) and/or modifying the product (e.g. via acetylation or phosphorylation) and/or forming a salt (e.g. a hydrochloride salt) of the product; and
- treating the product according to the method of the first aspect of the invention.

In some embodiments, the product is tryptophan. In some embodiments, the product may be a single isomer of tryptophan (e.g. L-tryptophan or D-tryptophan).

In some embodiments, the product is a modified amino acid, such as modified tryptophan e.g. N-acetyl tryptophan. In some embodiments, the product is *N*-acetyl-D,L-tryptophan (NADLT).

In some embodiments, the product is cystine (e.g. L-cystine or D-cystine). In some embodiments, the product is a salt of cystine (e.g. a hydrochloride salt, such as L-cystine dihydrochloride).

Thus, the invention also provides a method for preparing an amino acid or a nucleoside, the method comprising the method of the first aspect of the invention.

In some embodiments, the product which has been treated by the method of the invention meets one or more of the standards defined by the European pharmacopoeia 10.0.

For example, the European pharmacopoeia 10.0. requires that the appearance of a solution of N-acetyl tryptophan is clear and not more intensely coloured than reference solution Y₇ or GY7. Thus, in some embodiments wherein the product is *N*-acetyl-D,L-tryptophan (NADLT), the method results in a product which is no more intensely coloured than reference solution Y₇ or GY₇ according to the European pharmacopoeia 10.0. In some embodiments wherein the product is L-tryptophan, the method results in a product which is no more intensely coloured than reference solution BY₆ according to the European pharmacopoeia 10.0. In some embodiments wherein the product is L-cystine dihydrochloride, the method results in a product which is no more intensely coloured than reference solution Y₇ according to the European pharmacopoeia 10.0. The intensity of the solution of the product relative to the reference solution may be determined by eye.

Surprisingly, it has been found that propanol (e.g. 2-propanol) is particularly suitable for treating products according to the method of the invention. Treating products, in particular amino acids and nucleosides, with 2-propanol has been found to improve the appearance of the final product, producing a product that is whiter / less-coloured and, in some cases, more crystalline than products which have not been subjected to treatment. The use of 2-propanol is also thought to minimize or prevent microbial growth (bioburden) while the product is wet (waiting to be dried). As the skilled person will know, the term "bioburden" refers to the microbial cell count present in a product (e.g. bacterial, yeast or mould) and/or to the amount of endotoxins present (which is indicative of the presence of live or dead bacterial cells).

In addition, 2-propanol is particularly effective at removing water from the product of the preceding process, thereby reducing the drying time and/or temperature and enabling a more energy efficient process. Suitably, products such as amino acids and nucleosides have low solubility in 2-propanol, thereby minimizing product loss. These combined properties enable the preparation of products having an improved appearance and lower contaminants compared to prior art processes which rely on water alone. Advantageously, propanol also has lower toxicity than many other organic polar solvents, such as methanol.

Thus, the invention further provides the use of propanol (e.g. 2-propanol), subsequent to a chemical process for synthesising and/or purifying and/or modifying a product, and/or forming a salt of a product, for:
- decolouring the product;
- reducing the bioburden of the product and/or inhibiting the growth of microorganisms in the product; and/or
- improving the crystallinity of the product.

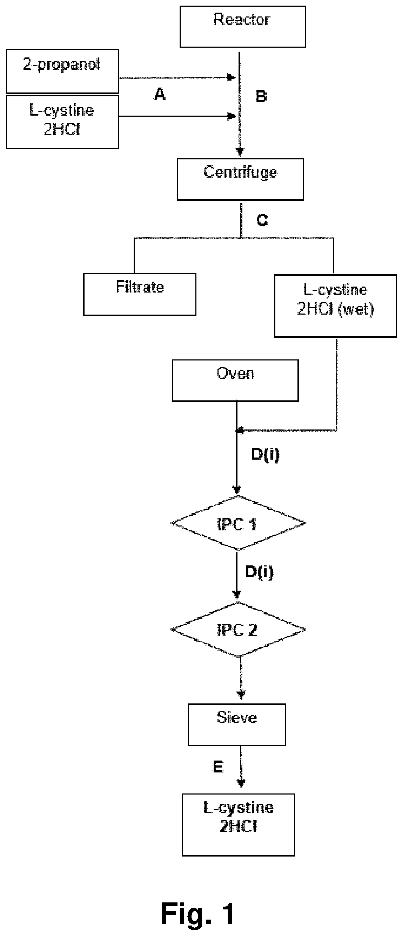

With reference to Figure 1, a process for treating L-cystine 2HC! comprises loading 2-propanol and wet L-cystine 2HC! into a reactor (step A). The L-cystine 2HC! may be the product of a prior chemical process, such as a process for converting L-cystine into L-cystine 2HCl. The amount of 2-propanol loaded into the reactor may be 1 kg propanol per kg of L-cystine. In the reactor, the 2-propanol and L-cystine 2HC! are cooled under stirring (step B). After stirring, the mixture is filtered by centrifugation in order to separate the L-cystine 2HC! from the 2-propanol filtrate (step C). The product L-cystine 2HC! is then loaded into an oven wherein it is dried in a first drying step (D(i)) at a first drying temperature. The moisture content (e.g. content of water and propanol) of the product is then determined (IPC1 (In Process Control)) and if it is found to be above a predetermined threshold, the first drying step is repeated. If the moisture content is below the predetermined threshold, a second drying step (D(ii)) is carried out at a second drying temperature, which is higher than the first drying temperature. The moisture content of the product is then determined (IPC2) and if it is found to be above a further predetermined threshold, the second drying step is repeated. If the moisture content is below the further predetermined threshold, the product is sieved (step E) to give the treated L-cystine 2HCl.

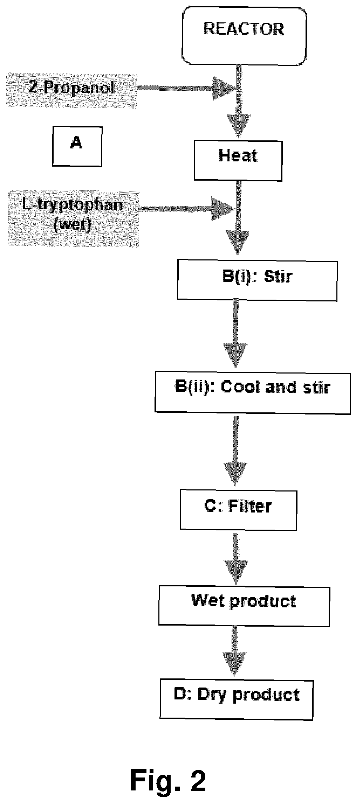

With reference to Figure 2, a process for treating L-tryptophan comprises loading 2-propanol and wet L-tryptophan into a reactor (step A). The L-tryptophan may be the product of a purification process, for example a recrystallisation process. The amount of 2-propanol loaded into the reactor may be 2.2 kg propanol per kg of L-tryptophan. In the reactor, the 2-propanol and L-tryptophan are heated to a first stirring temperature and stirred in a first stirring step (step B(i)). The reaction mixture is then cooled to a second stirring temperature and stirred in a second stirring step (step B(ii)). After stirring, the mixture is filtered by centrifugation in order to separate the L-tryptophan from the 2-propanol filtrate (step C). The product L-tryptophan is then loaded into an oven wherein it is dried (step D).

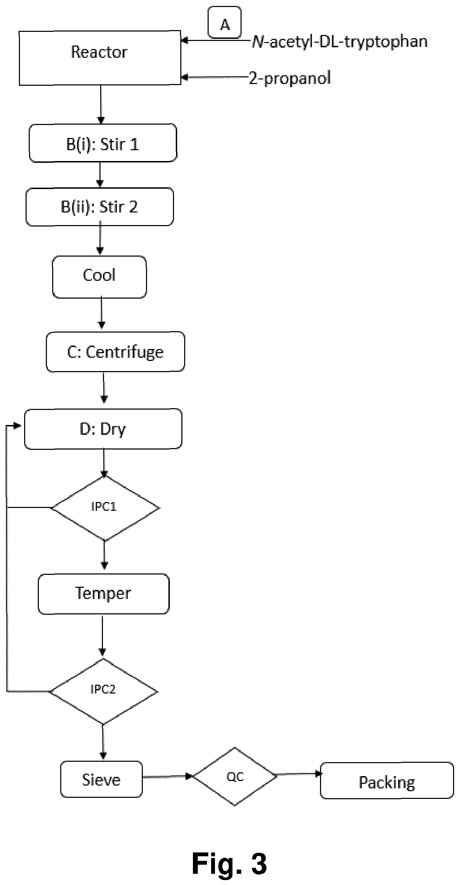

With reference to Figure 3, a process for treating *N*-acetyl-DL-tryptophan (NADLT) comprises loading 2-propanol and wet NADLT into a reactor (step A). The *N*-acetyl-DL-tryptophan may be the product of a synthesis process, for example a process for synthesising NADLT from L-tryptophan and acetic anhydride. The amount of 2-propanol loaded into the reactor may be 3 kg propanol per kg of NADLT. In the reactor, the 2-propanol and NADLT are heated to a first stirring temperature and stirred for a first period in a first stirring step (step B(i)). The reaction mixture is then cooled to a second stirring temperature and stirred for a second period in a second stirring step (step B(ii)), wherein the second period is shorter than the first period. The reaction mixture is then further cooled. After cooling, the mixture is filtered by centrifugation in order to separate the NADLT from the 2-propanol filtrate (step C). The product NADLT is then loaded into an oven where it is dried (step D). The moisture content of the product is then determined (IPC1) and if the moisture content of the NADLT is found to be above a predetermined threshold, the drying step (D) is repeated. If the product is dry, it is tempered (i.e. allowed to equilibrate, without active heating or cooling) to room temperature. After tempering, the moisture content of the product is then determined (IPC2) and if the NADLT is found to be not dry, the drying step (D) is repeated. If the product is dry, it is sieved and then, following quality control, packed.

### Example 1: NADLT

### Methodology

### Synthesis of NADLT

NADLT was synthesised by reaction of L-tryptophan and acetic anhydride based on the method described in J. Biol. Chem. 1932, 96:511-517.

### Treatment of NADLT with 2-propanol

2.1 L of 2-propanol was loaded to a 5 L glass reactor. The wet NADLT was loaded in portions ensuring the complete dispersion of the solid. Once loaded, the reactor jacket was set to 60 ºC and the mixture was stirred for 30 minutes at that temperature. The reactor jacket was then programmed to decrease the temperature to 15 ºC. Once that temperature was reached, the reaction mixture was stirred for 1 hour. After that, the reactor was discharged into a Büchner funnel where the product was filtered thoroughly. Finally the wet product was loaded into glass trays and put in an oven at 40 ºC for 16 h and then at 80 ºC for a further 2 hours. 505 g (60% overall yield) of NADLT was obtained as a white powder.

### Preparation of standard colour solutions

Standard solutions Y₇ and GY₇ were prepared as described in the European Pharmacopoeia 10.0 (chapter 2.2.2). All reagents used are reagent grade in accordance with the European Pharmacopoeia 10.0. The primary solutions are made as follows:
*Yellow solution.* Dissolve 46 g ferric chloride in about 900 mL of a mixture of 25 mL of hydrochloric acid and 975 mL water and dilute to 1000.0 mL with the same mixture. Titrate and adjust the solution to contain 45.0 mg of FeCl₃·6H₂O per millilitre by adding the same acidic mixture. Protect the solution from light.

Titration. Place in a 250 mL conical flask fitted with a ground-glass stopper, 10.0 mL of the solution, 15 mL of water, 5 mL of hydrochloric acid and 4 g of potassium iodide, close the flask, allow to stand in the dark for 15 minutes and add 100 mL of water. Titrate the liberated iodine with 0.1 M sodium thiosulfate, using 0.5 mL of starch solution, added towards the end of the titration, as indicator.

1 mL of 0.1 M sodium thiosulfate is equivalent to 27.03 mg of FeCl₃·6H₂O.

*Red solution.* Dissolve 60 g of cobalt chloride in about 900 mL of a mixture of 25 mL of hydrochloric acid and 975 mL of water and dilute to 1000.0 mL with the same mixture. Titrate and adjust the solution to contain 59.5 mg of CoCl₂·6H₂O per millilitre by adding the same acidic mixture.

Titration. Place in a 250 mL conical flask fitted with a ground-glass stopper, 5.0 mL of the solution, 5 mL of dilute hydrogen peroxide solution and 10 mL of a 300 g/L solution of NaOH. Boil gently for 10 min, allow to cool and add 60 mL of dilute sulfuric acid and 2 g of potassium iodide. Close the flask and dissolve the precipitate by shaking gently. Titrate the liberated iodine with 0.1 M sodium thiosulfate, using 0.5 mL of starch solution, added towards the end of the titration, as indicator. The end-point is reached when the solution turns pink.

1 mL of 0.1 M sodium thiosulfate is equivalent to 23.79 mg of COCl₂·6H₂O.

*Blue solution.* Dissolve 63 g copper sulfate pentahydrate in about 900 mL of a mixture of 25 mL HCI and 975 mL water and dilute to 1000.0 mL with the same mixture. Titrate and adjust the solution to contain 62.4 mg of CuSO₄·5H₂O per millilitre by adding the same acidic mixture. Titration. Place in a 250 mL conical flask fitted with a ground-glass stopper, 10.0 mL of the solution, 50 mL of water, 12 mL of dilute acetic acid and 3 g of potassium iodide. Titrate the liberated iodine with 0.1 M sodium thiosulfate, using 0.5 mL of starch solution, added towards the end of the titration, as indicator. The end-point is reached when the solution slows a slight pale brown colour.

1 mL of 0.1 M sodium thiosulfate is equivalent to 24.97 mg of CuSO₄·5H₂O.

Using the three primary solutions above, the Y and GY standard solutions are prepared as shown in Table 1:

**Table 1**

| | **Volume (mL)** | | | |
|---|---|---|---|---|
| **Standard solution** | **Yellow** | **Red** | **Blue** | **HCl (10 g/L HCl)** |
| Y (yellow) | 2.4 | 0.6 | 0.0 | 7.0 |
| GY (greenish yellow) | 9.6 | 0.2 | 0.2 | 0.0 |
| BY (brownish yellow) | 2.4 | 1.0 | 0.4 | 6.2 |

Using the standard solutions, the reference solutions are made as shown in Table 2:

**Table 2**

| **Reference solution** | **Standard solution (mL)** | **HCl (10 g/L HCl)** |
|---|---|---|
| Y₇ | solution Y (2.5) | 97.5 |
| GY₇ | solution GY (0.75) | 99.25 |
| BY₆ | solution BY (5.0) | 95.0 |

### Results

The NADLT following treatment with 2-propanol was white in colour (as observed by eye) and had a crystalline appearance. In contrast, commercial NADLT was off-white in colour.

Solutions of the NADLT treated with 2-propanol according to a method of the invention, and of commercially obtained NADLT, were prepared in 1% NaOH and compared with the standards Y₇ and GY₇.

Both of the NADLT solutions were less coloured than the standard solutions. However, the solution of commercial NADLT was slightly coloured, whereas the NADLT treated with 2-propanol according to the invention was colourless.

### Example 2: L-cystine dihydrochloride

### Methodology

### Preparation of L-cystine 2HCl

L-cystine dihydrochloride was prepared according to the method described in J. Am. Chem. Soc. 1958, 80: 3835-3838.

### Treatment of L-cystine 2HCl with 2-propanol

Wet L-cystine 2HC! was loaded into a crystallizer reactor together with 2-propanol at a ratio of 1 kg 2-propanol per kg of L-cystine 2HCl. The mixture was cooled to 15 °C under stirring for 30-60 minutes. The mixture was then centrifuged, and the filtered L-cystine 2HC! was placed in an oven where it was dried at 50 °C for 5 hours and then at 85 °C for 1 hour. The dry L-cystine 2HC! was sieved through a 1 mm sieve to give the final product.

### Results

The L-cystine 2HC! following treatment with 2-propanol was white in colour (as observed by eye) and had a crystalline appearance. In contrast, the non-treated L-cystine 2HC! was off-white to slightly yellow in colour, and was substantially amorphous (non-crystalline).

Solutions of the L-cystine 2HC! treated with 2-propanol according to a method of the invention, and of the non-treated L-cystine 2HCl, were prepared in 10% HCI and compared with the standard Y₇.

The solution of the product treated with 2-propanol was substantially colourless, and less coloured than the standard. The solution of the product which had not been treated with 2-propanol was slightly yellow in colour, and more coloured than the standard.

### Example 2: L-tryptophan

### Methodology

### Preparation of L-tryptophan

L-tryptophan was purchased from a commercial supplier and recrystallised using a process based on the method described in US5057615.

### Treatment of L-tryptophan with 2-propanol

Wet L-tryptophan (about 400 g) was loaded into a 5 litre reactor with 0.88 kg 2-propanol. The mixture was heated to 60 °C and stirred for 1 hour. The mixture was then cooled to 20 °C and stirred for a further 30 minutes. The product was filtered using a Büchner funnel and then dried in an oven for 16 hours at 40 ºC.

### Results

The L-tryptophan following treatment with 2-propanol was off-white in colour (as observed by eye) and had a crystalline appearance. In contrast, commercial samples of L-tryptophan were significantly more coloured, having a colour of from slightly yellow to light brown, and were less crystalline.

## Claims

1. A method of treating a product of a chemical process, the method comprising:
- adding the product to propanol in a vessel to obtain a mixture;
- stirring the mixture;
- filtering the mixture so as to separate the product from the propanol;
- drying the filtered product.

2. The method of claim 1, wherein the propanol is 2-propanol.

3. The method of claim 1 or claim 2, wherein the product is an amino acid, a nucleoside or a salt and/or dimer thereof.

4. The method of any one of claims 1 to 3, wherein the chemical process comprises a process for synthesising and/or purifying and/or modifying the product, and/or forming a salt of the product.

5. The method of any preceding claim, wherein the propanol is added to the vessel in an amount of from 0.5 kg to 5 kg 2-propanol per kg of product.

6. The method of any preceding claim, wherein stirring is carried out at a temperature of from 5 to 80 °C.

7. The method of claim 6, wherein stirring comprises a first stirring step which is carried out at a first temperature, and a second stirring step which is carried out at a second temperature, wherein the second temperature is lower than the first temperature.

8. The method of any preceding claim, wherein drying comprises a first drying step which is carried out at a first temperature, and a second drying step which is carried out at a second temperature, wherein the second temperature is higher than the first temperature.

9. The method of claim 8, wherein the first drying step is carried out for a greater period of time than the second drying step.

10. A method of preparing a product, the method comprising:
- synthesising, purifying and/or modifying the product, and/or forming a salt of the product; and
- treating the product according to the method of any one of claims 1 to 9.

11. The method of claim 10, wherein the product is an amino acid, a nucleoside or a salt or dimer thereof.

12. The use of propanol, subsequent to a chemical process for synthesising, purifying and/or modifying a product and/or for forming a salt of the product, for decolouring the product, optionally wherein the propanol is 2-propanol.

13. The use of propanol, subsequent to a chemical process for synthesising, purifying and/or modifying a product and/or for forming a salt of the product, for improving the crystallinity of the product, optionally wherein the propanol is 2-propanol.

14. The use of propanol, subsequent to a chemical process for synthesising, purifying and/or modifying a product and/or for forming a salt of the product, for reducing the bioburden of the product and/or inhibiting the growth of microorganisms in the product, optionally wherein the propanol is 2-propanol.

15. The use according to any one of claims 12 to 14, wherein the product is an amino acid, a nucleoside or a salt or dimer thereof.
